# FASCICULE DE BREVET EUROPEEN

(11) **EP 4 259 825 B1**
(45) Date de publication et mention de la délivrance du brevet: **20.08.2025**
(21) Numéro de dépôt: 21830663.7
(22) Date de dépôt: 07.12.2021
(51) Int. Cl.: C12Q 1/6851, C12Q 1/689

(54) **METHODE ET KIT DE DETECTION DE CHRISTENSENELLA MINUTA**
VERFAHREN UND KIT ZUM NACHWEIS VON CHRISTENSENELLA MINUTA
METHOD AND KIT FOR DETECTING CHRISTENSENELLA MINUTA

(30) Priorité: 08.12.2020 FR 2012821
(43) Date de publication de la demande: 18.10.2023
(73) Titulaire: Verb Biotics, Boston, MA 02210 (US)
(72) Inventeur: RAWADI, Georges, 75011 Paris (FR); CLAUS, Sandrine, Paule, 33200 Bordeaux (FR); TUDELA, Héloïse, 33150 Cenon (FR); DEJEAN, Guillaume, 33640 Arbanats (FR)
(74) Mandataire: Aquinov
(86) Numéro de dépôt international: PCT/EP2021/084521
(87) Numéro de publication internationale: WO 2022/122705

(56) Documents cités:
- VASANA JINATHAM ET AL: "Comparison of Gut Microbiota between Lean and Obese Adult Thai Individuals", HAN'GUG MI'SAENGMUL SAENGMYEONG GONG HAGHOEJI; KOREAN JOURNAL OF MICROBIOLOGY AND BIOTECHNOLOGY, vol. 46, no. 3, 28 September 2018 (2018-09-28), KR, pages 277 - 287, XP055679065, ISSN: 1598-642X, DOI: 10.4014/mbl.1711.11003
- ZOU YUANQIANG ET AL: "Taxonomic description and genome sequence of Christensenella intestinihominis sp. nov., a novel cholesterol-lowering bacterium isolated from human gut", BIORXIV, 14 November 2020 (2020-11-14), XP055829017, Retrieved from the Internet <URL:https://www.biorxiv.org/content/10.1101/2020.11.14.383166v1.full.pdf> [retrieved on 20210730], DOI: 10.1101/2020.11.14.383166
- ARNOLD JASON W. ET AL: "Intra-species Genomic and Physiological Variability Impact Stress Resistance in Strains of Probiotic Potential", FRONTIERS IN MICROBIOLOGY, vol. 9, 20 February 2018 (2018-02-20), XP055829006, DOI: 10.3389/fmicb.2018.00242
- CHANDRAN ARCHANA ET AL: "Relative expression of bacterial and host specific genes associated with probiotic survival and viability in the mice gut fed withLactobacillus plantarumLp91", MICROBIOLOGICAL RESEARCH, vol. 168, no. 9, 29 May 2013 (2013-05-29), pages 555 - 562, XP028703270, ISSN: 0944-5013, DOI: 10.1016/J.MICRES.2013.04.010
- EMBL-EBI: "CP029256.1 Christensenella minuta strain DSM 22607 chromosome, complete genome.", 1 January 2012 (2012-01-01), XP055829082, Retrieved from the Internet <URL:https://www.ebi.ac.uk/ena/browser/view/CP029256> [retrieved on 20210730]
- M. MOROTOMI ET AL: "Description of Christensenella minuta gen. nov., sp. nov., isolated from human faeces, which forms a distinct branch in the order Clostridiales, and proposal of Christensenellaceae fam. nov.", INTERNATIONAL JOURNAL OF SYSTEMATIC AND EVOLUTIONARY MICROBIOLOGY, vol. 62, no. 1, 25 February 2011 (2011-02-25), GB, pages 144 - 149, XP055232975, ISSN: 1466-5026, DOI: 10.1099/ijs.0.026989-0

## Description

### Domaine technique

L'invention concerne une méthode *in vitro* pour détecter ou diagnostiquer la présence d'au moins une bactérie de l'espèce *Christensenella minuta* chez un sujet, en détectant au moins une portion d'une séquence spécifique du génome de référence *Christensenella minuta.* La présente invention se rapporte également à des méthodes et kits permettant d'identifier, et suivre l'évolution de *C*. *minuta* chez un sujet.

### Etat de l'art

Notre microbiote intestinal ou « flore intestinale » est constitué d'un ensemble de bactéries, virus, parasites et champignons non pathogènes, soit 10¹² à 10¹⁴ micro-organismes présents dans le tube digestif de chaque individu, ce qui représente 2 à 10 fois plus que le nombre de cellules présent dans notre corps.

En 2012, un microbiologiste a identifié et cultivé à partir de fèces humaines une nouvelle espèce *: Christensenella minuta,* appartenant aux Clostridiales gram-négatifs. (Morotomi et al., Description of Christensenella minuta gen. nov., sp. nov., isolated from human faeces, which forms a distinct branch in the order Clostridiales, and proposal of Christensenellaceae fam. nov., International Journal of Systematic and Evolutionary Microbiology (2012), 62, 144-149).

En 2014, Goodrich et al. ont identifié *Christensenella minuta* comme le taxon bactérien le plus héréditaire chez l'homme et ont également suggéré son potentiel thérapeutique.

A ce jour, il est admis par la communauté scientifique que le microbiote intestinal humain est lié à l'apparition de certaines pathologies dites « non transmissibles », ce qui définit une dysbiose du microbiote.

L'écologie microbienne est alors de plus en plus étudiée ces dernières années en tant que régulateur des fonctions métaboliques et pour identifier son rôle central dans l'établissement d'un écosystème sain, en symbiose avec son hôte.

Certaines bactéries du genre *Christensenellaceae* ont ainsi été associées à un indice de masse corporelle bas dans de nombreuses cohortes humaines et d'autres études ont suggéré un rôle protecteur dans la régulation de l'inflammation.

Une absence ou une carence des bactéries du genre *Christensenellaceae* seraient notamment impliquées dans l'obésité, les maladies métaboliques, les maladies cardiaques et vasculaires, les maladies du foie et des canaux biliaires, du rein, les maladies articulaires liées au surpoids, les cancers en particulier les cancers liés au métabolisme et/ou à la dysbiose du microbiote, les maladies auto-immunes, les maladies dermatologiques atopiques, les maladies inflammatoires chroniques de l'intestin, les pneumonies, les diarrhées infectieuses, les allergies alimentaires, les néphrologies inflammatoires et les maladies neurologiques dont les maladies dégénératives ou les maladies neuropsychiatriques telles que les désordres liés à l'anxiété ou les désordres alimentaires (par ex. la boulimie).

La détection et l'identification d'un germe d'origine bactérienne est classiquement réalisée par des méthodes dites conventionnelles, telles que la mise en culture d'échantillon biologique, l'examen microscopique après coloration, l'hémoculture, la détection d'antigènes bactérien ou la détection par spectrométrie de masse tel que MALDI-TOF. Bien que ces méthodes soient majoritairement utilisées en routine en clinique et permettent l'identification du germe bactérien, elles nécessitent une culture d'au moins 24 à 48h de culture bactérienne pure, c'est-à-dire une culture comprenant un seul germe. Or, cela n'est possible que si la bactérie est cultivable et si l'échantillon ne contient pas un grand nombre de germes distincts. Ainsi, dans certains cas, un diagnostic plus rapide, indépendant des conditions de culture et à partir d'un échantillon plus complexe (présence de plusieurs germes) est requis.

Le diagnostic moléculaire permet également l'identification d'un germe bactérien et repose sur l'extraction et l'amplification d'un fragment de gène spécifique puis sa détection, en particulier un fragment d'ADN 16S ou un fragment d'ADN ribosomal. Les techniques moléculaires sont connues pour être très sensibles et spécifiques mais nécessitent une extraction de qualité afin de réduire notamment les risques de contamination réduisant la qualité de l'amplification. De plus, son coût est encore important et elles nécessitent que le fragment d'intérêt soit accessible et spécifique au germe bactérien d'intérêt. Or, de nombreuses espèces de bactéries possèdent un ADN 16S très proche qui surévalue la présence desdites bactéries dans un échantillon, telles que les bactéries de l'espèce *C*. *minuta.* Vasana Jinatham et al. (2018) et Zou Yuanqiang et al. (2020) décrivent un procédé moléculaire d'identification de C. minuta qui repose sur l'extraction et l'amplification d'un fragment de gène ADN 16S.

Arnold Jason et al. (2018) et Chandran Archana et al. (2013) décrivent des méthodes d'amplification du gène BSH par RT-qPCR pour détecter différentes souches de bactéries probiotiques.

Il existe donc un besoin clinique pour une nouvelle méthode d'identification des bactéries de l'espèce *C*. *minuta* qui soit simple, efficace, spécifique, rapide, sensible et économique. C'est l'objectif de la présente invention.

### Résumé de l'invention

Pour répondre à cet objectif, l'invention propose une méthode *in vitro* pour détecter la présence d'au moins une bactérie *Christensenella minuta* dans un échantillon grâce à la détection d'une séquence spécifique située entre les bornes 1,921,147 pdb et 2,014,152 pdb du génome de référence *C*. *minuta* déposée sous le numéro GenBank CP029256.1.

Par « pdb » ou « paire de base » au sens de l'invention, on entend l'appariement de deux bases nucléiques situées sur deux brins complémentaires d'ADN ou ARN.

Sur l'ensemble du génome de référence comprenant près de 3 millions de pdb, les inventeurs ont pu identifier une portion spécifique située entre les bornes 1,921,147 pdb et 2,014,152 pdb. Cette portion est particulièrement adaptée pour identifier, détecter, diagnostiquer de façon spécifique la présence d'au moins une bactérie de l'espèce *C*. *minuta.* C'est l'objet de la présente invention.

Ainsi, l'invention concerne une méthode *in vitro* pour détecter la présence d'au moins une bactérie *Christensenella minuta* dans un échantillon, ladite méthode comprenant la détection de la séquence SEQ ID NO : 1 située entre les bornes 1,921,147 pdb et 2,014,152 pdb du génome de référence *C*. *minuta* déposée sous le numéro GenBank CP029256.1. Une telle portion évite également de surévaluer l'abondance de l'espèce *C*. *minuta* dans l'échantillon cible. Préférentiellement, ladite méthode comprend la détection d'au moins 70 pdb de la séquence SEQ ID NO : 1.

Avantageusement, la séquence d'intérêt que l'on cherche à détecter est située dans le gène de la Bile Salt Hydrolase (BSH) situé entre les bornes 1,933,575 pdb et 1,934,552 pdb de SEQ ID NO: 2 dudit génome de référence.

Selon un autre aspect, l'invention concerne une méthode pour détecter/diagnostiquer une maladie ou un sujet susceptible de développer un trouble tel qu'une maladie et/ou un dysfonctionnement et/ou un déséquilibre, ledit trouble peut-être, par exemple une dysbiose intestinale, l'obésité ou une maladie inflammatoire.

L'invention concerne également une méthode pour surveiller l'évolution de la quantité de *C*. *minuta* chez un sujet, c'est-à-dire l'évolution de la quantité de *C*. *minuta* dans le microbiote intestinal d'un sujet, ladite méthode comprenant :
a) la mise en œuvre d'une méthode pour déterminer et quantifier la présence de *C*. *minuta* selon l'invention, dans un échantillon biologique dudit sujet, à un temps (t0)
b) la mise en œuvre d'une méthode pour déterminer et quantifier la présence de *C*. *minuta* selon l'invention, dans un échantillon biologique dudit sujet, à un temps (t1), et
c) comparer la quantité mesurée en a) à la quantité mesurée en b).

L'invention se rapporte aussi à une méthode pour déterminer ou adapter un régime thérapeutique ou alimentaire destiné à prévenir ou lutter contre une dysbiose intestinale en *C. minuta* et/ou une carence en *C*. *minuta* chez un sujet, ladite méthode comprenant :
a) la mise en œuvre d'une méthode pour déterminer un niveau ou une quantité de *C*. *minuta* selon l'invention, dans un échantillon biologique dudit sujet, et
b) la comparaison de celle-ci avec un niveau ou une quantité de *C*. *minuta,* dans un échantillon biologique dudit sujet après administration d'un traitement au sujet,
c) adapter/modifier le régime thérapeutique ou alimentaire dudit sujet sur la base de la comparaison des étapes a) et b).

Enfin, la présente demande se rapporte à des paires d'amorces spécifiques correspondant aux amorces de SEQ ID NO: 6 et SEQ ID NO: 7 et/ou les amorces de SEQ ID NO: 8 et SEQ ID NO: 9 et/ou les amorces de SEQ ID NO: 10 et SEQ ID NO: 11 permettant de cibler la SEQ ID NO: 2. Préférentiellement, lesdites paires d'amorces permettent de cibler respectivement les séquences SEQ ID NO: 3 et SEQ ID NO: 4 et SEQ ID NO: 5 du gène de la BSH.

L'invention concerne également un kit comprenant au moins une paire d'amorces choisie parmi les paires d'amorces citées précédemment et éventuellement au moins une sonde correspondant à la SEQ ID NO: 12 ou SEQ ID NO: 13 ou SEQ ID NO :15. Un tel kit est apte à mettre en œuvre l'une des méthodes selon l'invention.

D'autres caractéristiques et avantages ressortiront de la description détaillée de l'invention, des exemples et des figures qui vont suivre.

### Brève description des Figures

La Figure 1 est une représentation circulaire du génome de *Christensenella minuta* DSM 22607 comprenant la localisation d'une portion conservée parmi l'espèce *Christensenella minuta.*
La Figure 2A représente les résultats d'amplification par qPCR (Syber) du gène de la BSH, en particulier la gamme étalon pour les paires d'amorces de SEQ ID NO: 6 et SEQ ID NO: 7.
La Figure 2B représente les résultats d'amplification par qPCR (Syber) du gène de la BSH, en particulier la courbe de fusion pour les paires d'amorces de SEQ ID NO: 6 et SEQ ID NO: 7.
La Figure 2C représente les résultats d'amplification par qPCR (Syber) du gène de la BSH, en particulier la gamme étalon pour les paires d'amorces de SEQ ID NO: 8 et SEQ ID NO: 9.
La Figure 2D représente les résultats d'amplification par qPCR (Syber) du gène de la BSH, en particulier la courbe de fusion pour les paires d'amorces de SEQ ID NO: 8 et SEQ ID NO: 9.
La Figure 3 représente les résultats d'amplification par qPCR (Syber) de la bactérie *C*. *minuta 1* en UFC/g de matière fécale dans des échantillons enrichis ou non avec une quantité de C. *minuta 1* de référence pour les paires d'amorce de SEQ ID NO: 6 et SEQ ID NO: 7 (Figure 3A) et pour les paires d'amorce de SEQ ID NO: 8 et SEQ ID NO: 9 (Figure 3B).

### Description détaillée de l'invention

### Définition

Par « sujet » au sens de l'invention, on entend un mammifère, préférentiellement un humain ou un animal. Le sujet humain peut-être sain, c'est-à-dire n'ayant aucun symptôme de dysbiose intestinale ou de carence en *C*. *minuta,* ou peut être susceptible de développer ou suspecter de souffrir d'une dysbiose intestinale ou d'une carence en *C*. *minuta.*

Par « échantillon biologique » au sens de l'invention, on entend des échantillons susceptibles de contenir des bactéries, en particulier des bactéries de l'espèce *C*. *minuta,* par exemple des échantillons de fèces, de selles, de biopsie de colon ou des effluents de colon.

Par « évolution de la quantité de *C*. *minuta* » au sens de l'invention, on entend une augmentation ou une diminution de la quantité de *C*. *minuta* dans un échantillon, préférentiellement un échantillon biologique.

Par « amorces » au sens de l'invention, on entend des molécules d'acide nucléique isolées qui peuvent s'hybrider ou s'hybrider spécifiquement à des régions 5' ou 3' d'une séquence cible complémentaire. En général, elles ont une longueur d'environ 10 à 30 nucléotides et s'hybrident aux deux extrémités d'une région contenant environ 50 à 200 nucléotides, préférentiellement 70 nucléotides. Dans des conditions appropriées et avec des réactifs appropriés, ces amorces permettent l'amplification d'une molécule d'acide nucléique comprenant la séquence de nucléotides flanquée des amorces. Comme elles doivent être utilisées par paires, elles sont souvent appelées « paire d'amorces » ou « ensemble d'amorces » (par exemple SEQ ID NO: 6-7 ; SEQ ID NO: 8-9, SEQ ID NO: 10-11).

Par « sondes » au sens de l'invention, on entend des molécules capables de s'hybrider spécifiquement à une séquence d'intérêt (par exemple, de SEQ ID NO: 3 ou SEQ ID NO: 4 ou SEQ ID NO: 5). Elles sont utiles pour mettre en évidence la présence de ladite séquence d'intérêt dans les échantillons biologiques. Ces sondes peuvent comprendre au moins un nucléotide non naturel, par exemple un acide nucléique peptidique (PNA), un acide nucléique peptidique ayant un groupe phosphate (PHONA), un acide nucléique ponté ou un acide nucléique verrouillé (BNA ou LNA), et un acide nucléique morpholino. Les nucléotides non naturels comprennent également les acides nucléiques chimiquement modifiés ou les analogues d'acides nucléiques tels que l'ADN ou l'ARN de type méthylphosphonate, l'ADN ou l'ARN de type phosphorothioate, l'ADN ou l'ARN de type phosphoramidate, et 2'-O-méthyl-ADN ou ARN.

### Méthode

La présente invention a donc pour objet une méthode *in vitro* pour détecter dans un échantillon la présence d'au moins une bactérie *Christensenella minuta,* ladite méthode comprenant la détection d'au moins 70 pdb de la séquence SEQ ID NO: 1 située entre les bornes 1,921,147 pdb et 2,014,152 pdb du génome de référence *C*. *minuta* déposée sous le numéro GenBank CP029256.1.

La souche de référence de *Christensenella minuta* a été déposée auprès de l'Institut Leibniz DSMZ (Deutsche Sammlung von Mikroorganismen und Zellkulturen GmbH, en français Collection allemande de micro-organismes et de cultures cellulaires GmbH) sous le numéro DSM 22607 et son génome est accessible sous le numéro CP029256.1 de GenBank.

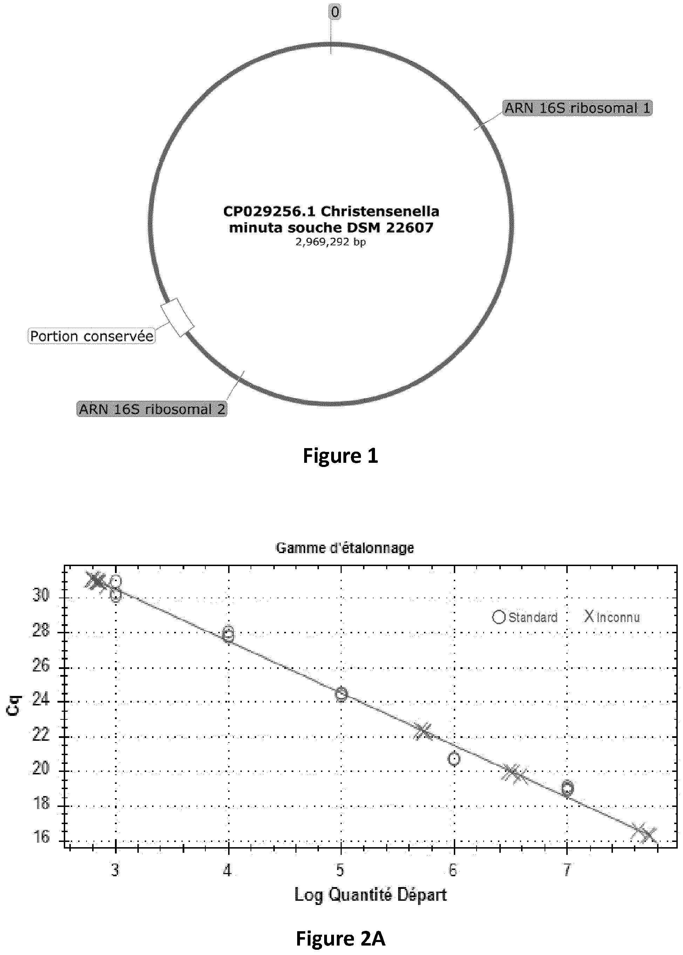

Ledit génome a été visualisé grâce à SnapGene Viewer (version 5.2). Le début du génome a ainsi été indiqué en 0, la position signalée en 0 étant la position de la première base de la séquence du fichier FASTA du génome. Le gène codant pour l'ARN ribosomal 16S 1 est situé entre les positions 468 243 et 468 318 (locus tag B1H56_02250) et le gène codant pour l'ARN ribosomal 16S 2 est situé entre les positions 1 735 562 et 1 737 097 (locus tag B1H56_08210) ont également été indiqués. La position de la portion d'ADN conservée se situe entre les nucléotides 1,921,147 et 2,014,152 (cf. Figure 1).

Cette portion d'ADN du génome *Christensenella minuta* DSM22607 a été déterminée, grâce à des analyses bio-informatiques, comme particulièrement conservée chez un certain nombre de souches de bactéries de cette espèce. Les résultats qui seront présentés ci-après permettent de démontrer son intérêt à des fins d'identification, de détection, de diagnostic et de quantification de cette bactérie dans des échantillons complexes, en utilisant des techniques comme l'amplification par PCR d'un fragment de cette portion.

Une séquence particulièrement adaptée dans le contexte de la présente invention est la séquence SEQ ID NO : 2. Elle située entre les bornes 1,933,575 pdb et 1,934,552 pdb correspondant au gène de la BSH et est présente dans la séquence conservée de SEQ ID NO: 1. Selon un mode de réalisation, la méthode selon l'invention comprend donc la détection d'au moins 70 pdb de la séquence SEQ ID NO : 2. Selon une variante, il peut s'agir de la détection de toute la séquence SEQ ID NO : 2.

Le gène de la BSH code pour une protéine impliquée dans la déconjugaison des acides biliaires. Ce gène n'est présent que chez certaines bactéries procaryotes. Le gène de la BSH de *Christensenella minuta* est unique tant par sa séquence nucléotidique que par la séquence d'acides aminés pour laquelle il code. Il est situé entre les bornes 1,933,575 pdb et 1,934,552 pdb du génome de référence *C*. *minuta* déposée sous le numéro GenBank CP029256.1. Ce gène a pour identifiant GenBank le numéro AYH40638.1. La séquence de SEQ ID NO: 2 donne de très bonnes performances d'amplification pour le gène de la BSH présent chez l'ensemble des bactéries appartenant à l'espèce *Christensenella minuta.*

**[Tableau 1]**

| | |
|---|---|
| SEQ ID NO: 2 | ATGTGTACAGCAATAACGTATTATACAAAGGATCACTATTTTGGAAGAAATCTG GATCTGGAATTTTCTTATAATGAAACAGTAACGGTTACACCAAAATATTATCCGT TTCATTTTCGTAATGGAAAAGTGCTCAATCATCATTATGCAATGATAGGGATGGC TTATATAGTTGATGACTTTCCGCTTTACTACGATGCAACAAATGAAAAGGGTCTC AGTATGGCAGGACTGAACTTCCCGGATAATGCGGACTATAAAGAAGTGAAGGA AGGATACGATAATATTGCTCCCTTTGAATTTATTCCATGGATACTCGGTCAATGT GCAAGTGTGAGTGAAGCAAGAATTCTCTTAGAACAGATTAACTTGGTAAATTTG AACTTTAGTGAAGAGCTGCCGCTTTCTCCATTACATTGGATGATTTCTGACCAGC GAGATTCCATTGTAGTAGAATCAACAAAAGATGGTTTGAAAGTTTTCGAAAACC CTGTAGGAGTTCTGACAAATAATCCTACCTTTGACTATCAGATGTTTAATCTCAAT AATTATATGCATTTATCGAAAGAACCCCCTGCAAATACATTTGCTGCCGAATTGG AACTTGAACAGTATAGCCGGGGTATGGGGGCGATAGGCCTTCCCGGAGATTTAT CATCCGCTTCGAGATTCGTGAAAGCAGCGTTTACAAAGATGAACTCGGTTTCCG GTGATTCGGAATCGGAAAGTATCAGCCAGTTTTTTCACATTCTTGGCTCGGTAGA GCAGCAACGAGGCTGCGTACATCTTGGTGAAGATAAATATGAAATAACAATTTA TTCTTCCTGCTGTAACATGGACAAGGGAATTTATTACTATACAACTTATGAAAAC AACCAGATCACAGCAGTTGATATGTATAAAGAAAATCTGGACGGAAACACTATA ATAAGCTATCCGCTTATGAAAGAACAGCAGATTAACTATCGGAATTACTAA |

De manière particulièrement préférée, une séquence d'intérêt spécifique de *C*. *minuta* est la SEQ ID NO: 3 et/ou la SEQ ID NO: 4 et/ou la SEQ ID NO: 5. Ces séquences sont en effet selon l'invention particulièrement adaptées pour identifier une bactérie appartenant à l'espèce *C*. *minuta.*

Ainsi, selon un mode de réalisation particulièrement préféré, la méthode selon l'invention vise à détecter la présence dans un échantillon d'au moins une bactérie *Christensenella minuta,* ladite méthode comprenant préférentiellement la détection de la séquence SEQ ID NO: 3 et/ou la SEQ ID NO: 4 et/ou la SEQ ID NO: 5.

Le tableau 2 ci-après décrit lesdites séquences d'intérêt.

**[Tableau 2]**

| | |
|---|---|
| SEQ ID NO: 3 | CTCGGTCAATGTGCAAGTGTGAGTGAAGCAAGAATTCTCTTAGAACAG ATTAACTTGGTAAATTTGAACTTTAGTGAAGAGCTGCCGCTTTCTCCATT ACATTGGATGATTTCTGACCAGCGAGATTCC |
| SEQ ID NO: 4 | AAGAACCCCCTGCAAATACATTTGCTGCCGAATTGGAACTTGAACAGTA TAGCCGGGGTATGGGGGCGATAGGCCTTCCCGGAGATTTATCATCCGC TTCGAGATTCGTGAAAGCAGCGTT |
| SEQ ID NO: 5 | TGCTCCCTTTGAATTTATTCCATGGATACTCGGTCAATGTGCAAGTGTGA GTGAAGCAAGAATTCTCTTAGAACAGATTAACTTGG |

Dans le contexte de l'invention, l'échantillon dans lequel on recherche la présence de *C. minuta* est préférentiellement un échantillon biologique d'un sujet. Selon une variante, l'échantillon peut également être un échantillon de sol, de sédiment, de nourriture, d'eau, un médicament ou des probiotiques. Un échantillon de sol, de sédiment, de nourriture ou d'eau peut ainsi être utilisé à des fins de recherche d'une éventuelle contamination. La recherche de la présence d'une bactérie dans un médicament ou des probiotiques est également d'intérêt à des fins de contrôle qualité.

Lorsque l'échantillon est un échantillon biologique, l'échantillon est préférentiellement choisi parmi les fèces, les selles, les biopsies du colon et les effluents du colon d'un sujet.

Dans le contexte de l'invention, le sujet peut être un humain ou un mammifère, préférentiellement un animal. Ainsi, la méthode selon l'invention est d'intérêt pour la médecine humaine et/ou vétérinaire.

La méthode vise donc à détecter une séquence spécifique appartenant à une bactérie *C. minuta* et ainsi identifier la présence d'une telle bactérie dans un échantillon donné, que ce soit à des fins diagnostics, pour vérifier la contamination d'un échantillon tel qu'un produit agroalimentaire ou à des fins de contrôle qualité, par exemple d'un médicament ou d'un probiotique.

Diverses techniques sont bien connues de l'homme du métier et celui-ci sera en mesure de déterminer la technique la plus adaptée pour détecter la séquence d'intérêt selon l'invention dans un échantillon donné.

Préférentiellement, la détection de la séquence d'intérêt est déterminée par une méthode d'amplification moléculaire telle que la PCR et ses dérivés et/ou une méthode de séquençage telle que la NGS.

Lorsque qu'il s'agit d'une méthode d'amplification moléculaire, il peut s'agir d'une PCR (*Polymerase Chain Reaction* en Anglais ou Réaction de Polymérisation en Chaîne en Français) ou de ses dérivés.

Par PCR dérivé, on entend au sens de l'invention une variante de la PCR classique. Il peut ainsi s'agir d'une PCR dites multiplexe, d'une méta-PCR, d'une PCR emboitée, d'une PCR asymétrique, d'une PCR quantitative (qPCR) ou d'une PCR digitale en micro-compartiments (dPCR).

Selon une variante, la détection de la séquence d'intérêt peut également être déterminée par séquençage telle que le Next Generation Sequencing ou NGS (en Français séquençage de prochaine génération).

Préférentiellement, la détection de la séquence d'intérêt est déterminée par PCR ou qPCR ou dPCR.

Selon un objet particulièrement préféré, la détection de la séquence est déterminée par PCR en utilisant les amorces de SEQ ID NO: 6 et SEQ ID NO: 7 et/ou les amorces de SEQ ID NO: 8 et SEQ ID NO: 9 et/ou les amorces de SEQ ID NO: 10 et SEQ ID NO: 11.

Lorsque la paire d'amorce est de SEQ ID NO: 6 et SEQ ID NO: 7, elle est apte à amplifier, la séquence d'intérêt SEQ ID NO: 1 ou SEQ ID NO: 2, préférentiellement la séquence d'intérêt SEQ ID NO: 3.

Lorsque la paire d'amorce est de SEQ ID NO: 8 et SEQ ID NO: 9, elle est apte à amplifier, la séquence d'intérêt SEQ ID NO: 1 ou SEQ ID NO: 2, préférentiellement la séquence d'intérêt SEQ ID NO: 4.

Lorsque la paire d'amorce est de SEQ ID NO: 10 et SEQ ID NO: 11, elle est apte à amplifier, la séquence d'intérêt SEQ ID NO: 1 ou SEQ ID NO: 2, préférentiellement la séquence d'intérêt SEQ ID NO: 5.

Lorsque, la détection de la séquence d'intérêt est déterminée par qPCR, la méthode comprend également des sondes de SEQ ID NO: 12 ou SEQ ID NO: 13 ou SEQ ID NO: 14.

Ainsi, la détection de la séquence d'intérêt est déterminée par qPCR en utilisant les paires d'amorce de SEQ ID NO: 6 et SEQ ID NO: 7 et la sonde de SEQ ID NO: 12 et/ou les paires d'amorce de SEQ ID NO: 8 et SEQ ID NO: 9 et la sonde de SEQ ID NO: 13 et/ou les paires d'amorce de SEQ ID NO: 10 et SEQ ID NO: 11 et la sonde de SEQ ID NO: 14.

Le tableau 3 ci-après décrit les séquences des amorces et sondes selon l'invention.

**[Tableau 3]**

| **Nom Amorce** | **Séquence 5'>3'** |
|---|---|
| SEQ ID NO: 6 | CTCGGTCAATGTGCAAGTGT |
| SEQ ID NO: 7 | GGAATCTCGCTGGTCAGAAA |
| SEQ ID NO: 12 | AGTGAAGAGCTGCCGCTTTCTCCATT |
| SEQ ID NO: 8 | AAGAACCCCCTGCAAATACA |
| SEQ ID NO: 9 | AACGCTGCTTTCACGAATCT |
| SEQ ID NO: 13 | ACTGTTCAAGTTCCAATTCGGCAGCAA |
| SEQ ID NO: 10 | TGCTCCCTTTGAATTTATTCC |
| SEQ ID NO: 11 | CCAAGTTAATCTGTTCTAAGAGA |
| SEQ ID NO: 14 | CACACTTGCACATTGACCGAGTATCCA |

Selon une variante, la détection et quantification de la bactérie C. *minuta* peut également être réalisée par un anticorps ou un aptamère reconnaissant spécifiquement une portion présente dans la séquence SEQ ID NO: 1 et/ou SEQ ID NO: 2 et/ou SEQ ID NO: 3 ou SEQ ID NO: 4 ou SEQ ID NO: 5.

Selon un autre objet, la méthode de l'invention comprend une étape de quantification des bactéries C. *minuta* détectées dans l'échantillon. La quantification des bactéries C. *minuta* présente dans un échantillon peut permettre d'utiliser la méthode selon l'invention à des fins diagnostic, suivi de patient, suivi de traitement, adapter un régime thérapeutique et/ou alimentaire.

Lorsque la méthode est utilisée à des fins diagnostic, elle permet d'identifier un sujet souffrant d'une maladie ou d'un déséquilibre ou d'un sujet susceptible de développer une maladie ou un déséquilibre.

Ainsi, l'invention se rapporte également à une méthode selon l'invention pour détecter/déterminer, préférentiellement une dysbiose intestinale en *C*. *minuta* et/ou une carence de *C*. *minuta* chez un sujet. Ledit sujet peut être sain ou malade.

Préférentiellement, la dysbiose intestinale en *C*. *minuta* et/ou la carence de *C*. *minuta* est détectée/déterminée chez un sujet, lorsque la quantité de bactérie *C*. *minuta* détectée est inférieure à 1%, préférentiellement à 0,4%, du microbiote dudit sujet. Le pourcentage est entendu en nombre de bactéries par rapport au nombre total de bactéries détectées. 1% est équivalent à 10⁹ CFU de C. *minuta* par gramme de fèces, le nombre total de bactéries admis étant en effet de 10¹¹ CFU/g de fèces. Aussi, un mode réalisation de l'invention est une méthode dans laquelle la quantité de bactérie *C*. *minuta* détectée est inférieure à 10⁹ CFU/g de fèces dudit sujet.

La méthode selon l'invention est préférentiellement apte à détecter/déterminer une maladie ou un sujet susceptible de développer une maladie, ladite maladie étant choisie de façon préférée parmi :
- Les maladies métaboliques, choisies parmi le diabète non-insulinodépendant, le diabète gestationnel, la NASH, la stéatose hépatique, la stéatose pancréatique, les hyperlipidémies, les hypercholestérolémie, l'infertilité liée au surpoids, l'incontinence urinaire liée au surpoids,
- Les autres maladies métaboliques chroniques, dont les thyroïdites,
- les maladies cardiaques et vasculaires, choisies parmi l'athérosclérose, les thrombopathies, les péricardites aiguës et la péricardite chronique constrictive, l'hypertension artérielle, les vascularites
- les maladies du foie et des canaux biliaires, choisies parmi les hépatites, la cirrhose biliaire primitive, la cholangite sclérosante primitive, la cirrhose, l'encéphalopathie hépatique, les lithiases vésiculaires
- les maladies articulaires liées au surpoids, choisies parmi l'ostéopénie, l'ostéoporose, l'arthrose, l'inflammation des disques vertébraux
- les maladies neurodégénératives, choisies parmi la maladie d'Alzheimer, la maladie de Parkinson, les maladies du motoneurone telles que la sclérose latérale amyotrophique, la sclérose latérale primitive et la maladie de Kennedy
- les cancers liés au métabolisme et/ou à une dysbiose du microbiote, choisies parmi les hépatocarcinomes, les cancers du tube digestif tel que de l'œsophage, de l'estomac et cancer colorectal, carcinome pancréatique, les tumeurs neuroendocrines (TNE) du système gastro-entéro-pancréatique, les tumeurs hépatiques, les tumeurs de la vésicule biliaire et des voies biliaires, les tumeurs rénales, les glioblastomes, les lymphomes, les myélomes multiples, la leucémie myéloïde chronique, les maladies myéloprolifératives chroniques, les carcinomes pulmonaires
- les maladies auto-immunes, choisies parmi le diabète insulino-dépendant, la polyarthrite rhumatoïde, la spondylarthrite ankylosante, le rhumatisme psoriasique, le lupus érythémateux disséminé, le syndrome auto-immun polyendocrinien
- les maladies dermatologiques atopiques, choisies parmi l'eczéma, le psoriasis
- les maladies inflammatoires chroniques de l'intestin, choisies parmi la maladie de Crohn, la recto-colite ulcéro-hémorragique, la diverticulite, l'œsophagite, gastrites, les pancréatites, l'ulcère gastro-duodénal, le syndrome du côlon irritable
- les troubles de la fonction respiratoire, choisies parmi l'asthme, la mucoviscidose, les maladies pulmonaires chroniques obstructives et bronchite chronique, les maladies pulmonaires interstitielles et fibroses pulmonaires, le syndrome d'apnée du sommeil (SAOS)
- les pneumonies, choisies parmi les pneumonies infectieuses, la pneumonie à influenza et la grippe aviaire, le syndrome respiratoire aigu sévère (SRAS), la pneumonie à pneumocystis
- Les diarrhées infectieuses, choisies parmi l'infection par Clostridium difficile, l'infection par EHEC, la gastro-entérite à salmonelles, l'entérite à campylobacter, les toxiinfections alimentaires par bactéries productrices d'entérotoxines, le choléra, la yersiniose, la shigellose, la cryptosporidiose, la listériose
- Les allergies alimentaires, choisies parmi la maladie cœliaque, l'intolérance au lactose, le syndrome de malabsorption des sels biliaires
- Les néphrologies inflammatoires ou en lien avec une dysbiose du microbiote, choisies parmi l'urétrite, l'insuffisance rénale chronique, les urolithiases
- Autres désordres inflammatoires, choisies parmi la sclérose en plaque, la lymphangite
- Les maladies neurologiques en lien avec une dysbiose du microbiote, choisies parmi l'anorexie, la boulimie, la dépression, le syndrome bipolaire, l'autisme, la schizophrénie, le syndrome de Tourette.

Selon un autre objet, l'invention se rapporte à une méthode pour surveiller ou suivre l'évolution de la quantité de *C*. *minuta* chez un sujet, c'est-à-dire l'évolution de la quantité de *C. minuta* dans le microbiote d'un sujet, ladite méthode comprenant :
a) la mise en œuvre d'une méthode selon l'un des quelconques modes de réalisation précédents pour déterminer et quantifier la présence de *C*. *minuta,* dans un échantillon biologique dudit sujet, à un temps (t0),
b) la mise en œuvre d'une méthode selon l'un des quelconques modes de réalisation précédents pour déterminer et quantifier la présence de *C. minuta,* dans un échantillon biologique dudit sujet, à un temps (t1), et
c) comparer la quantité mesurée en a) à la quantité mesurée en b).

Le sujet peut souffrir d'une maladie ou d'un déséquilibre tel qu'une dysbiose intestinale et susceptible de développer une telle maladie ou déséquilibre.

Selon un autre objet, l'invention concerne une méthode pour déterminer ou adapter un régime thérapeutique ou alimentaire destiné à prévenir ou lutter contre une maladie ou une dysbiose intestinale en *C. minuta* chez un sujet, ladite maladie étant choisie parmi l'une des maladies précédemment citées et ladite méthode comprenant :
a) la mise en œuvre d'une méthode selon l'un des quelconques modes de réalisation précédents pour déterminer un niveau ou une quantité de *C. minuta,* dans un échantillon biologique dudit sujet, et
b) la comparaison de celle-ci avec un niveau ou une quantité de *C. minuta,* dans un échantillon biologique dudit sujet après administration d'un traitement au sujet,
c) adapter/modifier le régime thérapeutique ou alimentaire dudit sujet sur la base de la comparaison des étapes a) et b).

Préférentiellement, le traitement administré au sujet peut être un traitement médicamenteux, une intervention chirurgicale, un régime alimentaire.

En particulier, ledit traitement est efficace si le niveau ou la quantité de C. *minuta,* dans un échantillon biologique dudit sujet est supérieur ou augmenté par rapport au niveau ou à la quantité de *C*. *minuta* déterminée avant le traitement.

### Amorces, sondes et kit

Selon un autre aspect, la présente demande concerne également les amorces aptes à être utilisées dans la présente invention.

Ainsi, les amorces, en particulier les paires d'amorces sont aptes à cibler et amplifier spécifiquement l'une des séquences choisies parmi la SEQ ID NO : 1, la SEQ ID NO : 2 et la SEQ ID NO : 3, préférentiellement la séquence SEQ ID NO: 3 du gène de la BSH et/ou la séquence SEQ ID NO: 4 du gène de la BSH et/ou la séquence SEQ ID NO: 5 du gène de la BSH. Préférentiellement, les paires d'amorce sont constituées des amorces de SEQ ID NO: 6 et SEQ ID NO: 7 et/ou des amorces de SEQ ID NO: 8 et SEQ ID NO: 9 et/ou des amorces de SEQ ID NO: 10 et SEQ ID NO: 11.

Lorsque la paire d'amorces de SEQ ID NO: 6 et SEQ ID NO: 7 est utilisée, elle cible et amplifie la séquence SEQ ID NO: 3 du gène de la BSH. Lorsque la paire d'amorces de SEQ ID NO: 8 et SEQ ID NO: 9 est utilisée, elle cible et amplifie la séquence SEQ ID NO: 4 du gène de la BSH. Lorsque la paire d'amorces de SEQ ID NO: 10 et SEQ ID NO: 11 est utilisée, elle cible et amplifie la séquence SEQ ID NO: 5 du gène de la BSH.

Ainsi, le kit comprend au moins deux amorces ciblant spécifiquement la séquence SEQ ID NO: 3 du gène de la BSH et/ou la séquence SEQ ID NO: 4 et/ou la séquence SEQ ID NO: 5 du gène de la BSH, ledit kit comprenant les amorces de de SEQ ID NO: 6 et SEQ ID NO: 7 et/ou des amorces de SEQ ID NO: 8 et SEQ ID NO: 9 et/ou des amorces de SEQ ID NO: 10 et SEQ ID NO: 11.

Selon un autre objet, l'invention concerne un kit comprenant au moins une paire d'amorce (ou deux amorces), ladite(s) paire d'amorce est choisie parmi la paire d'amorces de SEQ ID NO: 6 et SEQ ID NO: 7, la paire d'amorces de SEQ ID NO: 8 et SEQ ID NO: 9, la paire d'amorces de SEQ ID NO: 10 et SEQ ID NO: 11 et leur mélange.

Préférentiellement, le kit peut comprendre également au moins une sonde ciblant spécifiquement la séquence SEQ ID NO: 3 du gène de la BSH et/ou la séquence SEQ ID NO: 4 du gène de la BSH et/ou la séquence SEQ ID NO: 5 du gène de la BSH, ledit kit comprenant au moins une sonde choisie parmi la sonde de SEQ ID NO: 12, SEQ ID NO: 13, SEQ ID NO: 14.

Lorsque la sonde est de SEQ ID NO: 12, elle est associée aux amorces de séquence SEQ ID NO: 6 et SEQ ID NO: 7. Lorsque la sonde est de SEQ ID NO: 13, elle est associée aux amorces de séquence SEQ ID NO: 8 et SEQ ID NO: 9. Lorsque la sonde est de SEQ ID NO: 13, elle est associée aux amorces de séquence SEQ ID NO: 10 et SEQ ID NO: 11.

Enfin, ledit kit selon l'invention est adapté pour la mise en œuvre d'une des méthodes selon l'invention.

L'invention est à présent illustrée par des exemples non limitatifs de compositions selon l'invention et par des résultats.

### Exemples

### Exemple 1

Essai d'amplification par qPCR (Syber) du gène de la BSH avec les amorces selon l'invention (SEQ ID NO: 6 et SEQ ID NO: 7 ; et SEQ ID NO: 8 et SEQ ID NO: 9). Un tel essai permet de démontrer l'efficacité de la quantification sur un échantillon comprenant uniquement des souches *C. minuta* et sur un échantillon comprenant un mélange complexe de différentes bactéries.

Les gammes étalons ont été réalisées sur des échantillons d'ADN génomiques extrait de matières fécales de souris et enrichis avec 10⁹ Unité Formant Colonies (UFC) de *Christensenella minuta 1.*

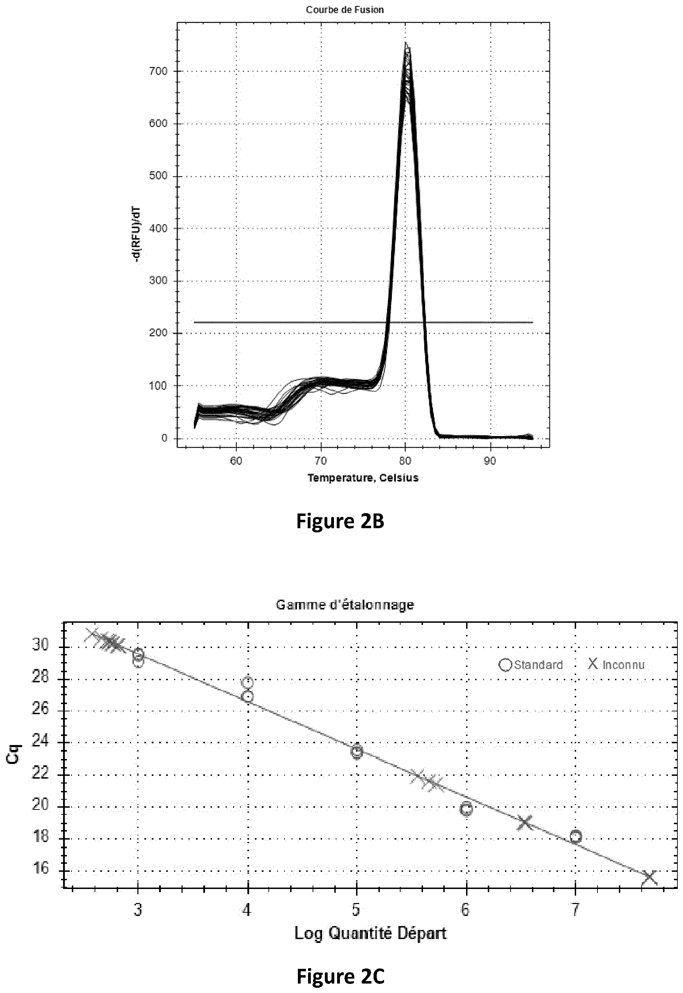

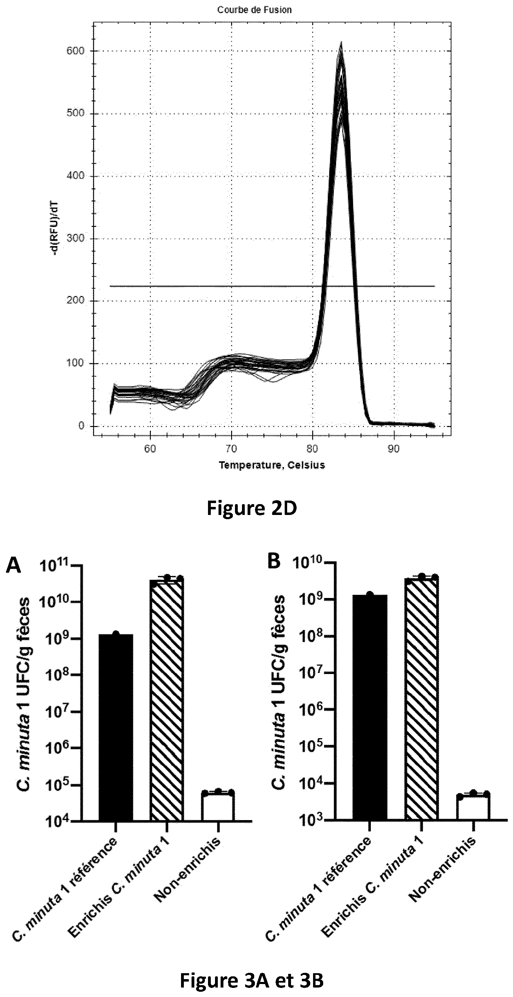

Les amorces ont été utilisées sur de l'ADN génomique extrait d'échantillons de matières fécales de souris (nourries avec un placebo). Ces échantillons ont été préalablement artificiellement enrichis avec 10⁹ Unité Formant Colonies (UFC) de *Christensenella minuta 1.* Cette matrice a ensuite été diluée en série par des facteurs de 10 à 10 millions de façon à obtenir une gamme étalon pour chaque paire d'amorce d'ADN et des réactions de PCR quantitative ciblant notre gène d'intérêt ont été réalisées (Figure 2A et 2C). Ces gammes étalons permettent de déterminer la linéarité, l'efficacité, la sensibilité et la reproductibilité de notre test. Ainsi, de remarquables scores d'efficacité (E) et des confiance (R²) ont été obtenus pour les amorces BSHqPCR2 et 3 sur les gammes étalons (respectivement E= 97,3% ; R²= 0,999 et E= 98,7% ; R²= 0,994). Les réactions de qPCR ont été réalisées avec Applied BiosystemsTM Power SYBRTM Green PCR Master Mix sur l'instrument CFX96 Touch Real-Time PCR Detection System (Bio-Rad). Les conditions du thermocycleur étaient les suivantes : une dénaturation initiale de 3 minutes at 98°C suivi de 40 cycles de 15 seconds à 98°C, et 15 secondes à 60°C. Une courbe de fusion a également été réalisée afin de vérifier la présence d'un seul produit PCR amplifié (Figure 2B et 2D). Trois réplicas techniques ont été réalisés pour chaque conditions.

### Exemple 2

Essai d'amplification par qPCR (Syber) du gène de la BSH avec les amorces selon l'invention (SEQ ID NO: 6 et SEQ ID NO: 7 ; et SEQ ID NO: 8 et SEQ ID NO: 9). Un tel essai permet de démontrer l'efficacité de la quantification sur un échantillon comprenant uniquement des souches *C. minuta* et sur un échantillon comprenant un mélange complexe de différentes bactéries.

Deux échantillons d'ADN génomique extraits de matières fécales de souris ont ensuite été utilisés afin de valider notre méthode de détection et quantification de la souche *Christensenella minuta 1:* un contrôle négatif non-enrichis en *C. minuta,* et un extrait référence préalablement enrichis avec une quantité déterminée de *C. minuta* (Figure 3A et 3B). Trois réplicas techniques ont été réalisés pour chaque conditions. La quantité de *C. minuta* exprimée en UFC/g de matière fécale a pu être déterminée en utilisant la gamme étalon. Ainsi, la quantification de *C*. *minuta* 1 dans l'échantillon « enrichis *C*. *minuta* 1 » reflète de manière robuste la quantité attendue, représentée par l'échantillon « *C. minuta* 1 référence. Les paires d'amorce de SEQ ID NO: 8 et SEQ ID NO: 9 démontrent de meilleurs résultats quant à la quantification de *Christensenella minuta* 1 au sein de cette matrice complexe (Figure 3B). En effet, le delta de quantification entre le nombre UFC/g attendu et le nombre retrouvé dans notre échantillon « enrichis *C. minuta* 1 » est plus robuste (Figure 3B).

## Revendications

1. Méthode *in vitro* pour détecter la présence d'au moins une bactérie *Christensenella minuta* dans un échantillon, ladite méthode comprenant la détection d'au moins 70 pdb de la séquence SEQ ID NO : 2 située entre les bornes 1,933,575 pdb et 1,934,552 pdb correspondant au gène de la BSH du génome de référence *C. minuta* déposée sous le numéro GenBank CP029256.1.

2. Méthode selon la précédente revendication, **caractérisée en ce que** l'échantillon est un échantillon biologique d'un sujet ou un échantillon de sol ou un médicament ou un probiotique.

3. Méthode selon la précédente revendication, **caractérisée en ce que** le sujet est un humain ou un animal.

4. Méthode selon la précédente revendication, dans laquelle la ladite méthode comprend la détection de la séquence SEQ ID NO: 3 et/ou la SEQ ID NO: 4 et/ou la SEQ ID NO : 5.

5. Méthode selon l'une des revendications 2 à 4, dans laquelle l'échantillon biologique est un échantillon choisi parmi les fèces, les selles, les biopsies du colon et les effluents du colon dudit sujet.

6. Méthode selon l'une des précédentes revendications, dans laquelle la détection de la séquence est réalisée par une méthode d'amplification moléculaire dites PCR et/ou par une méthode de séquençage.

7. Méthode selon l'une des précédentes revendications, dans laquelle la détection de la séquence est réalisée par PCR en utilisant les amorces de SEQ ID NO: 5 et SEQ ID NO: 6 et/ou les amorces de SEQ ID NO: 7 et SEQ ID NO: 8 et/ou les amorces de SEQ ID NO: 9 et SEQ ID NO: 10.

8. Méthode selon l'une des précédentes revendications, dans laquelle la méthode comprend une étape de quantification des bactéries *C. minuta* détectées dans l'échantillon.

9. Méthode selon l'une des revendications 2 à 8, pour détecter une dysbiose intestinale en *C. minuta* et/ou une carence de *C. minuta* chez un sujet.

10. Méthode selon la revendication précédente, dans laquelle la quantité de bactérie *C. minuta* détectée est inférieure à 10⁹ CFU/g de fèces dudit sujet.

11. Méthode selon l'une des revendications 9 ou 10, pour détecter une maladie ou un sujet susceptible de développer une maladie, ladite maladie est choisie parmi :
- Les maladies métaboliques, choisies parmi le diabète non-insulinodépendant, le diabète gestationnel, la NASH, la stéatose hépatique, la stéatose pancréatique, les hyperlipidémies, les hypercholestérolémie, l'infertilité liée au surpoids, l'incontinence urinaire liée au surpoids,
- Les autres maladies métaboliques chroniques, dont les thyroïdites,
- les maladies cardiaques et vasculaires, choisies parmi l'athérosclérose, les thrombopathies, les péricardites aiguës et la péricardite chronique constrictive, l'hypertension artérielle, les vascularites
- les maladies du foie et des canaux biliaires, choisies parmi les hépatites, la cirrhose biliaire primitive, la cholangite sclérosante primitive, la cirrhose, l'encéphalopathie hépatique, les lithiases vésiculaires
- les maladies articulaires liées au surpoids, choisies parmi l'ostéopénie, l'ostéoporose, l'arthrose, l'inflammation des disques vertébraux
- les maladies neurodégénératives, choisies parmi la maladie d'Alzheimer, la maladie de Parkinson, les maladies du motoneurone telles que la sclérose latérale amyotrophique, la sclérose latérale primitive et la maladie de Kennedy
- les cancers liés au métabolisme et/ou à une dysbiose du microbiote, choisies parmi les hépatocarcinomes, les cancers du tube digestif tel que de l'œsophage, de l'estomac et cancer colorectal, carcinome pancréatique, les tumeurs neuroendocrines (TNE) du système gastro-entéro-pancréatique, les tumeurs hépatiques, les tumeurs de la vésicule biliaire et des voies biliaires, les tumeurs rénales, les glioblastomes, les lymphomes, les myélomes multiples, la leucémie myéloïde chronique, les maladies myéloprolifératives chroniques, les carcinomes pulmonaires
- les maladies auto-immunes, choisies parmi le diabète insulino-dépendant, la polyarthrite rhumatoïde, la spondylarthrite ankylosante, le rhumatisme psoriasique, le lupus érythémateux disséminé, le syndrome auto-immun polyendocrinien
- les maladies dermatologiques atopiques, choisies parmi l'eczéma, le psoriasis
- les maladies inflammatoires chroniques de l'intestin, choisies parmi la maladie de Crohn, la recto-colite ulcéro-hémorragique, la diverticulite, l'œsophagite, gastrites, les pancréatites, l'ulcère gastro-duodénal, le syndrome du côlon irritable
- les troubles de la fonction respiratoire, choisies parmi l'asthme, la mucoviscidose, les maladies pulmonaires chroniques obstructives et bronchite chronique, les maladies pulmonaires interstitielles et fibroses pulmonaires, le syndrome d'apnée du sommeil (SAOS)
- les pneumonies, choisies parmi les pneumonies infectieuses, la pneumonie à influenza et la grippe aviaire, le syndrome respiratoire aigu sévère (SRAS), la pneumonie à pneumocystis
- Les diarrhées infectieuses, choisies parmi l'infection par Clostridium difficile, l'infection par EHEC, la gastro-entérite à salmonelles, l'entérite à campylobacter, les toxiinfections alimentaires par bactéries productrices d'entérotoxines, le choléra, la yersiniose, la shigellose, la cryptosporidiose, la listériose
- Les allergies alimentaires, choisies parmi la maladie cœliaque, l'intolérance au lactose, le syndrome de malabsorption des sels biliaires
- Les néphrologies inflammatoires ou en lien avec une dysbiose du microbiote, choisies parmi l'urétrite, l'insuffisance rénale chronique, les urolithiases
- Autres désordres inflammatoires, choisies parmi la sclérose en plaque, la lymphangite
- Les maladies neurologiques en lien avec une dysbiose du microbiote, choisies parmi l'anorexie, la boulimie, la dépression, le syndrome bipolaire, l'autisme, la schizophrénie, le syndrome de Tourette.

12. Méthode pour surveiller l'évolution de la quantité de *C. minuta* chez un sujet, ladite méthode comprenant :
a) la mise en œuvre d'une méthode selon l'une des revendications précédentes pour déterminer et quantifier la présence de *C. minuta,* dans un échantillon biologique dudit sujet, à un temps (t0)
b) la mise en œuvre d'une méthode selon l'une des revendications précédentes pour déterminer et quantifier la présence de *C. minuta,* dans un échantillon biologique dudit sujet, à un temps (t1), et
c) comparer la quantité mesurée en a) à la quantité mesurée en b).

13. Méthode pour déterminer ou adapter un régime thérapeutique ou alimentaire destiné à prévenir ou lutter contre une dysbiose intestinale en *C. minuta* chez un sujet, ladite méthode comprenant :
a) la mise en œuvre d'une méthode selon l'une des revendications 1 à 11 pour déterminer un niveau ou une quantité de *C. minuta,* dans un échantillon biologique dudit sujet, et
b) la comparaison de celle-ci avec un niveau ou une quantité de *C. minuta,* dans un échantillon biologique dudit sujet après administration d'un traitement au sujet,
c) adapter/modifier le régime thérapeutique ou alimentaire dudit sujet sur la base de la comparaison des étapes a) et b).

14. Kit comprenant au moins deux amorces ciblant spécifiquement la séquence SEQ ID NO: 3 du gène de la BSH et/ou la séquence SEQ ID NO: 4 et/ou la séquence SEQ ID NO: 5 du gène de la BSH, ledit kit comprenant les amorces de de SEQ ID NO: 6 et SEQ ID NO: 7 et/ou des amorces de SEQ ID NO: 8 et SEQ ID NO: 9 et/ou des amorces de SEQ ID NO: 10 et SEQ ID NO: 11.

15. Kit selon la revendication précédente comprenant également au moins une sonde ciblant spécifiquement la séquence SEQ ID NO: 3 du gène de la BSH ou la séquence SEQ ID NO: 4 du gène de la BSH ou la séquence SEQ ID NO: 5 du gène de la BSH, ledit kit comprenant les sondes de SEQ ID NO: 12 ou SEQ ID NO: 13 ou SEQ ID NO: 14.

## Patentansprüche

1. In-vitro-Verfahren zum Nachweisen des Vorhandenseins von mindestens einem *Christensenella minuta-Bakterium* in einer Probe, das Verfahren umfassend den Nachweis von mindestens 70 pdb der Sequenz SEQ ID NO: 2, die sich zwischen den Grenzen 1.933.575 pdb und 1.934.552 pdb befindet, die dem Gen der BSH des *C. minuta*-Referenzgenoms entsprechen, das unter der GenBank-Nummer CP029256.1 hinterlegt ist.

2. Verfahren nach dem vorstehenden Anspruch,
**dadurch gekennzeichnet, dass** die Probe eine biologische Probe eines Subjekts oder eine Bodenprobe oder ein Medikament oder ein Probiotikum ist.

3. Verfahren nach dem vorstehenden Anspruch,
**dadurch gekennzeichnet, dass** das Subjekt ein Mensch oder ein Tier ist.

4. Verfahren nach dem vorstehenden Anspruch, wobei das Verfahren den Nachweis der Sequenz SEQ ID NO: 3 und/oder SEQ ID NO: 4 und/oder SEQ ID NO: 5 umfasst.

5. Verfahren nach einem der Ansprüche 2 bis 4, wobei die biologische Probe eine Probe ist, die aus Fäkalien, Stühlen, Dickdarmbiopsien und Dickdarmausflüssen des Subjekts ausgewählt ist.

6. Verfahren nach einem der vorstehenden Ansprüche, wobei der Nachweis der Sequenz durch ein molekulares Amplifikationsverfahren, genannt PCR, und/oder durch ein Sequenzierungsverfahren erfolgt.

7. Verfahren nach einem der vorstehenden Ansprüche, wobei der Nachweis der Sequenz durch PCR unter Verwendung der Primer von SEQ ID NO: 5 und SEQ ID NO: 6 und/oder der Primer von SEQ ID NO: 7 und SEQ ID NO: 8 und/oder der Primer von SEQ ID NO: 9 und SEQ ID NO: 10 erfolgt.

8. Verfahren nach einem der vorstehenden Ansprüche, wobei das Verfahren einen Schritt für eine Quantifizierung der *C. minuta*-Bakterien umfasst, die in der Probe nachgewiesen werden.

9. Verfahren nach einem der Ansprüche 2 bis 8 zum Nachweisen einer C. minuta-Darmdysbiose und/oder eines Mangels an *C. minuta* in einem Subjekt.

10. Verfahren nach dem vorstehenden Anspruch, wobei die nachgewiesene Menge an *C*. *minuta-Bakterium* weniger als 10⁹ KBE/g Fäkalien des Subjekts beträgt.

11. Verfahren nach einem der Ansprüche 9 oder 10 zum Nachweisen einer Erkrankung oder eines Subjekts, das anfällig auf eine Entwicklung einer Erkrankung ist, wobei die Erkrankung ausgewählt ist aus:
- Stoffwechselerkrankungen, ausgewählt aus nicht insulinabhängigem Diabetes, Schwangerschaftsdiabetes, NASH, Leberverfettung, Pankreasverfettung, Hyperlipidämien, Hypercholesterinämien, Unfruchtbarkeit im Zusammenhang mit Übergewicht, Harninkontinenz im Zusammenhang mit Übergewicht,
- anderen chronischen Stoffwechselerkrankungen, darunter Thyreoiditis,
- Herz- und Gefäßerkrankungen, ausgewählt aus Arteriosklerose, Thrombopathien, akuten Perikarditiden und chronischer konstriktiver Perikarditis, arterieller Hypertonie, Vaskulitiden
- Leber- und Gallenwegserkrankungen, ausgewählt aus Hepatitiden, primärer biliärer Zirrhose, primärer sklerosierender Cholangitis, Zirrhose, hepatischer Enzephalopathie, Gallensteinen
- Gelenkerkrankungen im Zusammenhang mit Übergewicht, ausgewählt aus Osteopenie, Osteoporose, Arthrose, Bandscheibenentzündung
- neurodegenerativen Erkrankungen, ausgewählt aus Alzheimer-Krankheit, Parkinson-Krankheit, Motoneuron-Erkrankungen wie amyotrophe Lateralsklerose, primäre Lateralsklerose und Kennedy-Krankheit
- Krebserkrankungen im Zusammenhang mit dem Stoffwechsel und/oder einer Dysbiose der Mikrobiota, ausgewählt aus Leberkarzinomen, Krebserkrankungen des Verdauungstrakts wie Speiseröhren-, Magen- und Dickdarmkrebs, Pankreaskarzinom, neuroendokrinen Tumoren (NETs) des gastroenteropankreatischen Systems, Lebertumoren, Tumoren der Gallenblase und der Gallenwege, Nierentumoren, Glioblastomen, Lymphomen, multiplen Myelomen, chronischer myeloischer Leukämie, chronischen myeloproliferativen Erkrankungen, Lungenkarzinomen
- Autoimmunerkrankungen, ausgewählt aus insulinabhängigem Diabetes, rheumatoider Arthritis, ankylosierender Spondylitis, Psoriasis-Arthritis, systemischem Lupus erythematodes, polyendokrinem Autoimmunsyndrom
- atopischen dermatologischen Erkrankungen, ausgewählt aus Ekzemen, Psoriasis
- chronisch entzündlichen Darmerkrankungen, ausgewählt aus Morbus Crohn, Colitis ulcerosa, Divertikulitis, Ösophagitis, Gastritis, Pankreatitis, Magengeschwür, Reizdarmsyndrom
- Atemfunktionsstörungen, ausgewählt aus Asthma, Mukoviszidose, chronisch obstruktiven Lungenerkrankungen und chronischer Bronchitis, interstitiellen Lungenerkrankungen und Lungenfibrosen, Schlafapnoe-Syndrom (SAOS)
- Lungenentzündungen, ausgewählt aus infektiösen Lungenentzündungen, Influenza-Pneumonie und Vogelgrippe, schwerem akuten respiratorischen Syndrom (SARS), Pneumocystis-Pneumonie
- infektiösen Diarrhöen, ausgewählt aus Infektion durch Clostridium difficile, Infektion durch EHEC, Salmonellen-Gastroenteritis, Campylobacter-Enteritis, durch Enterotoxine hervorgerufenen Lebensmittelkrankheiten, Cholera, Yersiniose, Shigellose, Kryptosporidiose, Listeriose
- Nahrungsmittelallergien, ausgewählt aus Zöliakie, Laktoseintoleranz, Gallensalzmalabsorptionssyndrom
- entzündlichen Nephrologien oder im Zusammenhang mit einer Dysbiose der Mikrobiota, ausgewählt aus Urethritis, chronischem Nierenversagen, Urolithiasen
- anderen entzündlichen Erkrankungen, ausgewählt aus Multipler Sklerose, Lymphangitis
- neurologischen Erkrankungen im Zusammenhang mit einer Dysbiose der Mikrobiota, ausgewählt aus Anorexie, Bulimie, Depression, bipolarem Syndrom, Autismus, Schizophrenie und Tourette-Syndrom.

12. Verfahren zum Überwachen der Veränderung der Menge an *C. minuta* in einem Subjekt, das Verfahren umfassend:
a) Durchführen eines Verfahrens nach einem der vorstehenden Ansprüche zum Bestimmen und Quantifizieren des Vorhandenseins von *C. minuta* in einer biologischen Probe des Subjekts zu einem Zeitpunkt (t0)
b) Durchführen eines Verfahrens nach einem der vorstehenden Ansprüche zum Bestimmen und Quantifizieren des Vorhandenseins von *C. minuta* in einer biologischen Probe des Subjekts zu einem Zeitpunkt (t1), und
c) Vergleichen der in a) gemessenen Menge mit der in b) gemessenen Menge.

13. Verfahren zum Bestimmen oder Anpassen eines Therapie- oder Ernährungsplans, der dafür bestimmt ist, *C. minuta*-Darmdysbiose in einem Subjekt zu verhindern oder zu bekämpfen, das Verfahren umfassend:
a) Durchführen eines Verfahrens nach einem der Ansprüche 1 bis 11 zum Bestimmen eines Niveaus oder einer Menge an *C. minuta* in einer biologischen Probe des Subjekts, und
b) Vergleichen davon mit einem Niveau oder einer Menge an *C. minuta* in einer biologischen Probe des Subjekts nach Verabreichung einer Behandlung an das Subjekt,
c) Anpassen/Ändern des Therapie- oder Ernährungsplans des Subjekts basierend auf dem Vergleich der Schritte a) und b).

14. Kit, umfassend mindestens zwei Primer, die spezifisch auf die Sequenz SEQ ID NO: 3 des Gens der BSH und/oder die Sequenz SEQ ID NO: 4 und/oder die Sequenz SEQ ID NO: 5 des Gens der BSH abzielen, das Kit umfassend die Primer von SEQ ID NO: 6 und SEQ ID NO: 7 und/oder Primer von SEQ ID NO: 8 und SEQ ID NO: 9 und/oder Primer von SEQ ID NO: 10 und SEQ ID NO: 11.

15. Kit nach dem vorstehenden Anspruch, ferner umfassend mindestens eine Sonde, die spezifisch auf die Sequenz SEQ ID NO: 3 des Gens der BSH oder die Sequenz SEQ ID NO: 4 des Gens der BSH oder die Sequenz SEQ ID NO: 5 des Gens der BSH abzielt, das Kit umfassend die Sonden von SEQ ID NO: 12 oder SEQ ID NO: 13 oder SEQ ID NO: 14.

## Claims

1. An *in vitro* method for detecting the presence of at least one *Christensenella minuta* bacterium in a sample, said method comprising detecting at least 70 bp of the sequence SEQ ID NO: 2 located between the boundaries 1,933,575 bp and 1,934,552 bp corresponding to the BSH gene of the reference genome *C. minuta* filed under GenBank number CP029256.1.

2. The method according to the preceding claim, **characterized in that** the sample is a biological sample of a subject or a sample of soil or a medication or a probiotic.

3. The method according to the preceding claim, **characterized in that** the subject is a human or an animal.

4. The method according to the preceding claim, wherein said method comprises the detection of the sequence SEQ ID NO: 3 and/or SEQ ID NO: 4 and/or SEQ ID NO: 5.

5. The method according to one of claims 2 to 4, wherein the biological sample is a sample selected from feces, stools, colon biopsies and colon effluents of said subject.

6. The method according to one of the preceding claims, wherein the detection of the sequence is carried out by a molecular amplification method known as PCR and/or by a sequencing method.

7. The method according to one of the preceding claims, wherein the detection of the sequence is carried out by PCR using the primers of SEQ ID NO: 5 and SEQ ID NO: 6 and/or the primers of SEQ ID NO: 7 and SEQ ID NO: 8 and/or the primers of SEQ ID NO: 9 and SEQ ID NO: 10.

8. The method according to one of the preceding claims, wherein the method comprises a step of quantifying the *C. minuta* bacteria detected in the sample.

9. The method according to one of claims 2 to 8, for detecting *C. minuta* intestinal dysbiosis and/or a *C. minuta* deficiency in a subject.

10. The method according to the preceding claim, wherein the amount of C. *minuta* bacterium detected is less than 10⁹ CFU/g of feces of said subject.

11. The method according to one of claims 9 or 10, for detecting a disease or a subject likely to develop a disease, said disease is selected from:
- metabolic diseases, selected from non-insulin-dependent diabetes, gestational diabetes, NASH, hepatic steatosis, pancreatic steatosis, hyperlipidemia, hypercholesterolemia, infertility linked to excess weight, urinary incontinence linked to excess weight,
- other chronic metabolic diseases, including thyroiditis,
- cardiac and vascular diseases, selected from atherosclerosis, thrombopathies, acute pericarditis and chronic constrictive pericarditis, arterial hypertension, vasculitides
- liver and bile duct diseases, selected from hepatitises, primary biliary cirrhosis, primary sclerosing cholangitis, cirrhosis, hepatic encephalopathy, vesicular lithiases
- joint diseases linked to excess weight, selected from osteopenia, osteoporosis, osteoarthritis, vertebral disc inflammation
- neurodegenerative diseases, selected from Alzheimer's disease, Parkinson's disease, motor neuron diseases such as amyotrophic lateral sclerosis, primitive lateral sclerosis and Kennedy disease
- cancers linked to metabolism and/or to dysbiosis of the microbiota, selected from hepatocellular carcinomas, gastrointestinal tract cancers such as esophageal, stomach and colorectal cancer, pancreatic carcinoma, neuroendocrine tumors (NETs) of the gastrointestinal-pancreatic system, hepatic tumors, gallbladder and bile duct tumors, renal tumors, glioblastomas, lymphomas, multiple myelomas, chronic myeloid leukemia, chronic myeloproliferative diseases, lung carcinomas
- autoimmune diseases, selected from insulin-dependent diabetes, rheumatoid arthritis, ankylosing spondylitis, psoriatic arthritis, systemic lupus erythematosus, autoimmune polyendocrine syndrome
- atopic skin diseases, selected from eczema, psoriasis
- chronic inflammatory bowel diseases, selected from Crohn's disease, hemorrhagic ulcerative colitis, diverticulitis, esophagitis, gastritis, pancreatitides, gastrointestinal ulcers, irritable bowel syndrome
- respiratory function disorders, selected from asthma, cystic fibrosis, chronic obstructive pulmonary diseases and chronic bronchitis, interstitial lung diseases and pulmonary fibroses, sleep apnea syndrome (OSAS)
- pneumonias, selected from infectious pneumonias, influenza pneumonia and avian flu, severe acute respiratory syndrome (SARS), pneumocystis pneumonia
- infectious diarrhea, selected from Clostridium difficile infection, EHEC infection, salmonella gastroenteritis, campylobacter enteritis, food poisoning by enterotoxin-producing bacteria, cholera, yersiniosis, shigellosis, cryptosporidiosis, listeriosis
- food allergies, selected from celiac disease, lactose intolerance, bile salt malabsorption syndrome
- inflammatory kidney disorders or others related to dysbiosis of the microbiota, selected from urethritis, chronic renal failure, urolithiasis
- other inflammatory disorders, selected from multiple sclerosis, lymphangitis
- neurological diseases related to dysbiosis of the microbiota, selected from anorexia, bulimia, depression, bipolar syndrome, autism, schizophrenia, Tourette syndrome.

12. A method for monitoring the evolution of the amount of *C. minuta* in a subject, said method comprising:
a) implementing a method according to one of the preceding claims to determine and quantify the presence of *C. minuta,* in a biological sample of said subject, at a time (t0)
b) implementing a method according to one of the preceding claims to determine and quantify the presence of *C. minuta,* in a biological sample of said subject, at a time (t1), and
c) comparing the amount measured in a) to the amount measured in b).

13. A method for determining or adapting a therapeutic or dietary regimen intended to prevent or combat *C. minuta* intestinal dysbiosis in a subject, said method comprising:
a) implementing a method according to one of claims 1 to 11 to determine a level or an amount of *C. minuta* in a biological sample of said subject, and
b) comparing it with a level or an amount of *C. minuta* in a biological sample of said subject after administering treatment to the subject,
c) adapting/modifying the therapeutic or dietary regimen of said subject based on the comparison of steps a) and b).

14. A kit comprising at least two primers specifically targeting the sequence SEQ ID NO: 3 of the BSH gene and/or the sequence SEQ ID NO: 4 and/or the sequence SEQ ID NO: 5 of the BSH gene, said kit comprising the primers of SEQ ID NO: 6 and SEQ ID NO: 7 and/or primers of SEQ ID NO: 8 and SEQ ID NO: 9 and/or primers of SEQ ID NO: 10 and SEQ ID NO: 11.

15. The kit according to the preceding claim, also comprising at least one probe specifically targeting the sequence SEQ ID NO: 3 of the BSH gene or the sequence SEQ ID NO: 4 of the BSH gene or the sequence SEQ ID NO: 5 of the BSH gene, said kit comprising the probes of SEQ ID NO: 12 or SEQ ID NO: 13 or SEQ ID NO: 14.
